(19) Europäisches Patentamt
European Patent Office
Office européen des brevets

(11) Publication number: 0 176 369
A2

# (12) EUROPEAN PATENT APPLICATION

(21) Application number: 85306866.6

(22) Date of filing: 26.09.85

(51) Int. Cl.⁴: C 07 D 501/36
C 07 D 501/46, A 61 K 31/545
//C07D417/12

(30) Priority: 26.09.84 JP 201000/84

(43) Date of publication of application:
02.04.86 Bulletin 86/14

(84) Designated Contracting States:
DE FR GB IT

(71) Applicant: YAMANOUCHI PHARMACEUTICAL CO. LTD.
No. 5-1 Nihonbashi-Honcho, 2-chome Chuo-ku
Tokyo(JP)

(72) Inventor: Koda, Akio
No.4-5-3, Hibarigaoka Kita
Hoya-shi Tokyo(JP)

(72) Inventor: Shibanuma, Tadao
No.596-11, Oyaguchi
Urawa-shi Saitama(JP)

(72) Inventor: Yamazaki, Atsuki
No.2-7-8-506, Shimura
Itabashi-ku Tokyo(JP)

(72) Inventor: Nakano, Koji
No.1086-1, Oaza Sanegaya Shiraoka-cho
Minamisaitama-gu Saitama(JP)

(72) Inventor: Nagano, Noriaki
No.13-1, Ayase
Hasuda-shi Saitama(JP)

(72) Inventor: Maeda, Tetsuya
No.3-14-6, Yotsuya
Urawa-shi Saitama(JP)

(72) Inventor: Murakami, Yukiyasu
No.306-3, Omaki
Urawa-shi Saitama(JP)

(72) Inventor: Hara, Ryuchiro
No. 3-19-11, Honcho
Nakano-ku Tokyo(JP)

(74) Representative: Geering, Keith Edwin et al,
REDDIE & GROSE 16 Theobalds Road
London WC1X 8PL(GB)

## (54) 7-(Alpha-(4-oxo-2-azetidinyl(or -azetidinylalkyl)-oxyimino)-alpha-(2-amino-4-thiazolyl)acetamido[-3- substituted-Delta 3-cephem-4-carboxylic acid compounds and salts thereof, their production, and medicaments containing them.

(57) A cephalosporin compound of the following general formula (I), or a pharmacologically acceptable salt thereof

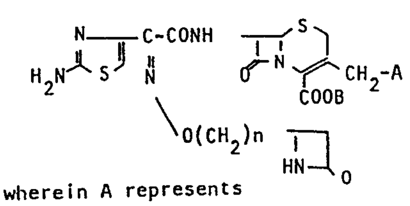 (I)

wherein A represents

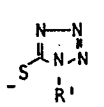 , 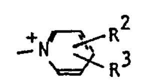 , 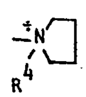 , or 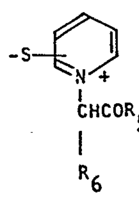

wherein $R^1$ represents a hydrogen atom or a $C_1$ to $C_5$ alkyl group which is unsubstituted or substituted by at least one hydroxyl group; $R^2$ and $R^3$ are the same or different and selected from an amino group, a hydrogen atom, $C_1$ to $C_5$ alkyl groups and $C_1$ to $C_5$ alkoxy groups; $R^4$ represents a $C_1$ to $C_5$ alkyl group; and $R^5$ and $R^6$ are the same or different and selected from a hydrogen atom and $C_1$ to $C_5$ alkyl groups; $-COOB$ represents $-COOH$ or $-COO^-$; and n represents 0, 1 or 2.

EP 0 176 369 A2

# 7-[α-(4-OXO-2-AZETIDINYL(OR -AZETIDINYLALKYL)-OXYIMINO)-α-(2-AMINO-4-THIAZOLYL)ACETAMIDO]-3-SUBSTITUTED-$\Delta^3$-CEPHEM-4-CARBOXYLIC ACID COMPOUNDS AND SALTS THEREOF, THEIR PRODUCTION, AND MEDICAMENTS CONTAINING THEM

This invention relates to antibacterial cephalosporin compounds, the production of these compounds, medical compositions containing them, and salts thereof.

The compounds of this invention are those of the general formula (I) and salts thereof:

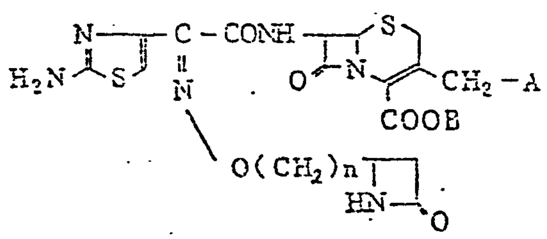

(I)

wherein A represents

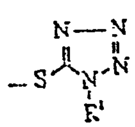 , 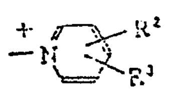 , 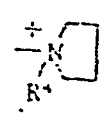 or 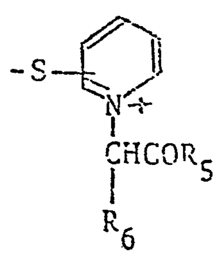

where $R^1$ represents a hydrogen atom or a lower alkyl group which is unsubstituted or substituted by a hydroxyl group(s); $R^2$ and $R^3$ are the same or different and each represents an amino group, a hydrogen atom, a lower alkyl group, or a lower alkoxy group; $R^4$ represents a lower alkyl group; and $R^5$ and $R^6$ are the same or different

BAD ORIGINAL

0176369

and each represents a hydrogen atom or a lower alkyl group; and -COOB represents -COOH or $-COO^-$; and n represents 0 or an integer of 1 or 2.

The term "lower" in the foregoing definition means a straight or branched carbon chain having 1 to 5 carbon atoms. Thus examples of the "lower alkyl" for the definitions of $R^2$, $R^3$, $R^4$, $R^5$ and $R^6$ are methyl, ethyl, propyl, isopropyl, butyl, isobutyl, tert-butyl, pentyl, neopentyl, tert-pentyl, etc. "A lower alkyl group which is unsubstituted or substituted by a hydroxyl group(s)" for the definition of $R^1$ means straight or branched carbon chain alkyl having 1 to 5 carbon atoms substitutable by one or more hydroxyl group(s); thus, as such unsubstituted or substituted/lower alkyl, there are hydroxymethyl,/2-hydroxyethyl, 3-hydroxybutyl, 4-hydroxybutyl, 5-hydroxypentyl, 2,4-dihydroxybutyl, etc. in addition to the groups listed as the examples of the "lower alkyl". Examples of "a lower alkoxy" for the definitions of $R^2$ and $R^3$ are methoxy, ethoxy, propoxy, isopropoxy, butoxy, isobutoxy, tert-butoxy, pentyloxy, neopentyloxy, tert-pentyloxy which belong to straight or branched carbon chain alkyloxy having 1 to 5 carbon atoms. Examples of "halogen" (hereinafter mentioned) are iodine, bromine, chlorine, etc. Substituents ($R^2$ and $R^3$) on the pyridinio group in the above formula I may exist at any position of the pyridinio group; and the -S- group in the

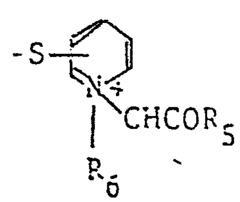

group may exist at any position of the pyridine ring.

3-Substituted cephalosporin compounds having a (2-oxo-3-pyrrolidinyl)oxy moiety in the 7-position substituent are known as described in, for example, EP 101,265 and EP 117,109.

BAD ORIGINAL

On the other hand, the compounds of the present invention have a 4-oxoazetidinyl-oxy(-methoxy or -ethoxy) moiety in the 7-position substituent. Thus, the present invention provides cephalosporin compounds having the characteristics that the substituent at the 3-position is a specific N-containing group (that is,

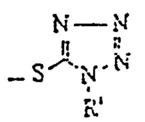 , 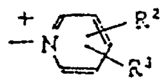 , 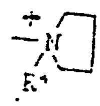 or 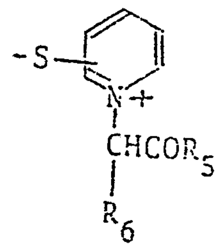 )

and the substituent at the 7-position is an α-(2-amino-4-thiazolyl)-α-(4-oxo-2-azetidinyl-substituted-oxyimino-acetyl group.

The salts of the cephalosporin compounds I include pharmaceutically acceptable nontoxic salts examples of which are salts of inorganic bases (e.g. of an alkali metal such as sodium or potassium or of an alkaline earth metal such as calcium or magnesium); ammonium salts; salts of organic bases such as trimethylamine, triethylamine, cyclohexylmaine, dicyclohexylamine, diethanolamine, or basic amino acids such as ornithine, arginine and lysine; salts of inorganic acids such as hydrochloric acid, hydrobromic acid, sulfuric acid, phosphoric acid; and salts of organic acids such as acetic acid, fumaric acid, citric acid, maleic acid, methanesulfonic acid, ethanesulfonic acid.

The compounds I with an iminoether-type oxime and 2-substituted thiazol group include geometrical isomers and tautomers; this invention includes all these syn-form and anti-form geometrical isomers and mutual tautomers

BAD ORIGINAL

Compounds of this invention show superior antibacterial activity against various pathogens including several important gram positive and negative pathogens, and thus are useful for medicaments (especially antibacterial agents), additives for feeds, and preservatives. Some of the compounds of this invention show antibacterial activity against methicilline-resistant bacteria.

Antibacterial activities (minimum effective inhibitory concentrations) of compounds of formula I (taken from the following Examples) are shown in the following Table.

Table (minimum effective inhibitory concentrations: μg/ml)

| Strain \ Compound | Example No. 1 | 2 | 4 | 7 |
|---|---|---|---|---|
| Staph. epidermidis IIb 866 | 0.2 | 0.78 | 0.2 | 0.2 |
| Sal. enteritidis 1891 | 0.025 | 0.1 | 0.1 | 0.05 |
| Kleb. pneumoniae ATCC 10031 | 0.025 | 0.05 | 0.1 | 0.05 |
| Serr. marcescens II D 620 | 0.05 | 0.1 | 0.1 | 0.1 |
| Ps. aeruginosa NCTC 10490 | 0.78 | 0.1 | 0.78 | 0.39 |

Antibacterial medicaments containing compounds according to the invention (free compounds and their salts) may be prepared by conventional methods using conventional carriers or excipients. They may for example be administered orally as tablets, pills, capsules, granules; parenterally by intravenous or intramuscular

injection; or as syrups or suppositories. Compounds of this invention have superior solubility in water, and aqueous solutions of high concentration can be produced. Thus compounds of this invention have practical value in that injection preparations such as intramuscular injections can be easily provided.

The appropriate dose is determined in each case considering factors such as the symptom, age and sex of the patient, and for an adult a daily total of 250 to 3,000 mg is usually administered in one to four doses.

The compounds of this invention can be produced by various processes. Typical production processes are explained hereinafter.

Process 1:

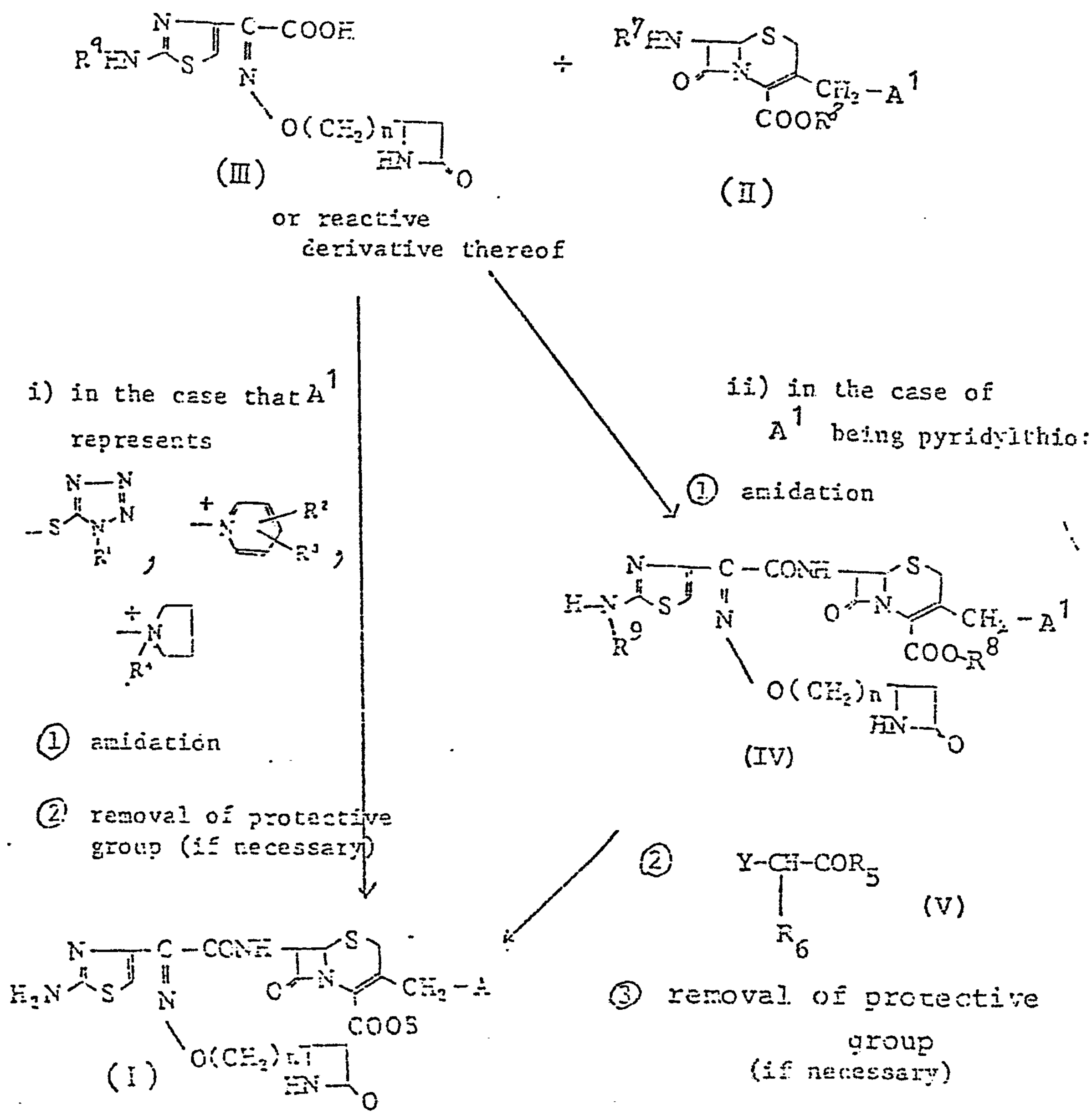


In the above formulae, $A^1$ represents

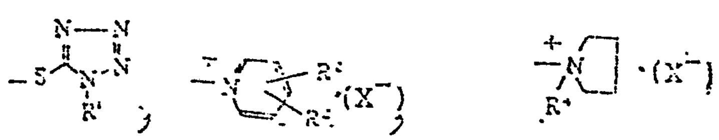

or a pyridylthio group, $R^7$ represents a hydrogen atom or a protective group for an amino group, $R^8$ represents a hydrogen atom or a protective group for a carboxyl group, and $R^9$ represents a hydrogen atom or

BAD ORIGINAL

a protective group for an amino group. In the above formulae, /n, A, B, $R^1$, $R^2$, $R^3$, $R^4$, $R^5$, and $R^6$ are as defined above. Y represent a halogen atom. $X^-$ represents an anion. Compounds I can thus be produced by reacting substituted oxyiminothiazolylacetic acid derivative (III) or reactive derivative thereof with 7-amino-3-cephem derivative (II), [and then, in the case of $A^1$ being a pyridylthio group, reacting the formed compound (IV) with compound (V)] and then, if necessary, releasing any protective group(s). More detailed explanation is made below.

i) In the case of $A^1$ being 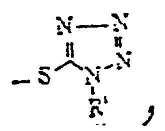 , 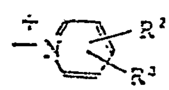 or 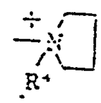 :

α-(4-oxo-2-azetidinyl-substituted-imino)thiazolylacetic acid derivative (III) or reactive derivative thereof is reacted with 3-substituted-$\Delta^3$-cephem-4-carboxylic acid derivative (II) followed, if necessary, by release of any protective group(s).

ii) In the case of $A^1$ being a pyridylthio group:

α-(oxo-2-azetidinyl-substituted-imino)thiazolylacetic acid derivative (III) or reactive derivative thereof

is reacted with 3-pyridylthiomethyl-$\Delta^3$-cephem-4-carboxylic acid derivative (II), and then the formed compound (IV) is reacted with halogen compound of formula

$$\begin{array}{l} Y\text{-}CH\text{-}COR_5 \\ \quad\;\; | \\ \quad\;\; R_6 \end{array} \qquad (V)$$

followed, if necessary, by release of any protective group(s)

to give the compound (I).

The reaction of compound (III) with compound (II) (that is, amidation reaction) is usually performed in a solvent under cooling at room temperature or below. Any solvents which do not take part in the reaction can be used. Examples of the solvent usually used are organic solvents such as dioxane, dichloroethane, ether, ethanol, methanol, ethyl acetate, tetrahydrofuran, acetone, anisole, chloroform, dimethylformamide; these solvents may be used alone or in appropriate combination.

A compound (III) may be a free carboxylic acid or a reactive derivative thereof. Suitable examples of the reactive derivative are mixed acid anhydrides, acid anhydrides, acid halides, active esters, active amides, acid azides. When using the compound (III) in the form of a free carboxylic acid, it is preferred to use a condensing agent such as N,N'-dicyclo-hexylcarbodiimide or N,N'-diethylcarbodiimide.

According to the kind of reactive derivative of carboxylic acid used, it may be preferred for smooth reaction to operate in the presence of a base. Examples of such a base are inorganic bases such as sodium hydrogencarbonate, potassium hydrogencarbonate, sodium carbonate, potassium carbonate; and organic bases such as trimethylamine, triethylamine, dimethylaniline, pyridine.

In this case the protective group for an amino group may be one usually used in the field of peptide chemistry and practical examples are acyl groups such as a formyl group, an acetyl group, a propionyl group, a tert-butoxycarbonyl group, a methoxyacetyl group, a methoxypropionyl group, a benzyloxycarbonyl group, a p-nitrobenzyloxycarbonyl group; tri-loweralkylsilyl groups such as a trimethylsilyl group; and aralkyl groups such as a benzyl group, a benzhydryl group (diphenylmethyl group), a trityl group.

Practical examples of the protective group for a carboxy group are those which can be released easily under mild conditions, such as a benzhydryl group, a $\beta$-methylsulfonylethyl group, a phenacyl group, a p-methoxybenzyl group, a tert-butyl group, a p-nitrobenzyl group; and lower-alkylsilyl groups such as trimethylsilyl group.

The reaction of 3-pyridinylthiomethyl-$\Delta^3$-cephem-4-carboxylic acid derivative (IV) with halogen compound (V) (in the case of $A_1$ being pyridylthio) is preferably performed in a solvent which does not take part in the reaction. Examples of the solvent usually used are organic solvents such dimethysulfoxide, dimethylformamide, benzene, toluene, chloroform, dichloromethane, ethyl acetate, ether, tetrahydrofuran, dioxane. An equivalent amount or an excess of compound (V) to compound (IV) is usually used in the reaction. The reaction is usually

performed at room temperature or under cooling.

The removal of a protecting group from the reaction product thus obtained is easily performed, when the group is tri-loweralkylsilyl, by contact with water; and when the group is benzhydryl, trityl, tert-butyl, formyl, etc., by using an acid such as formic acid, trifluoroacetic acid-anisole mixture, hydrobromic acid-acetic acid mixture, hydrochloric acid-dioxane mixture.

Process 2:

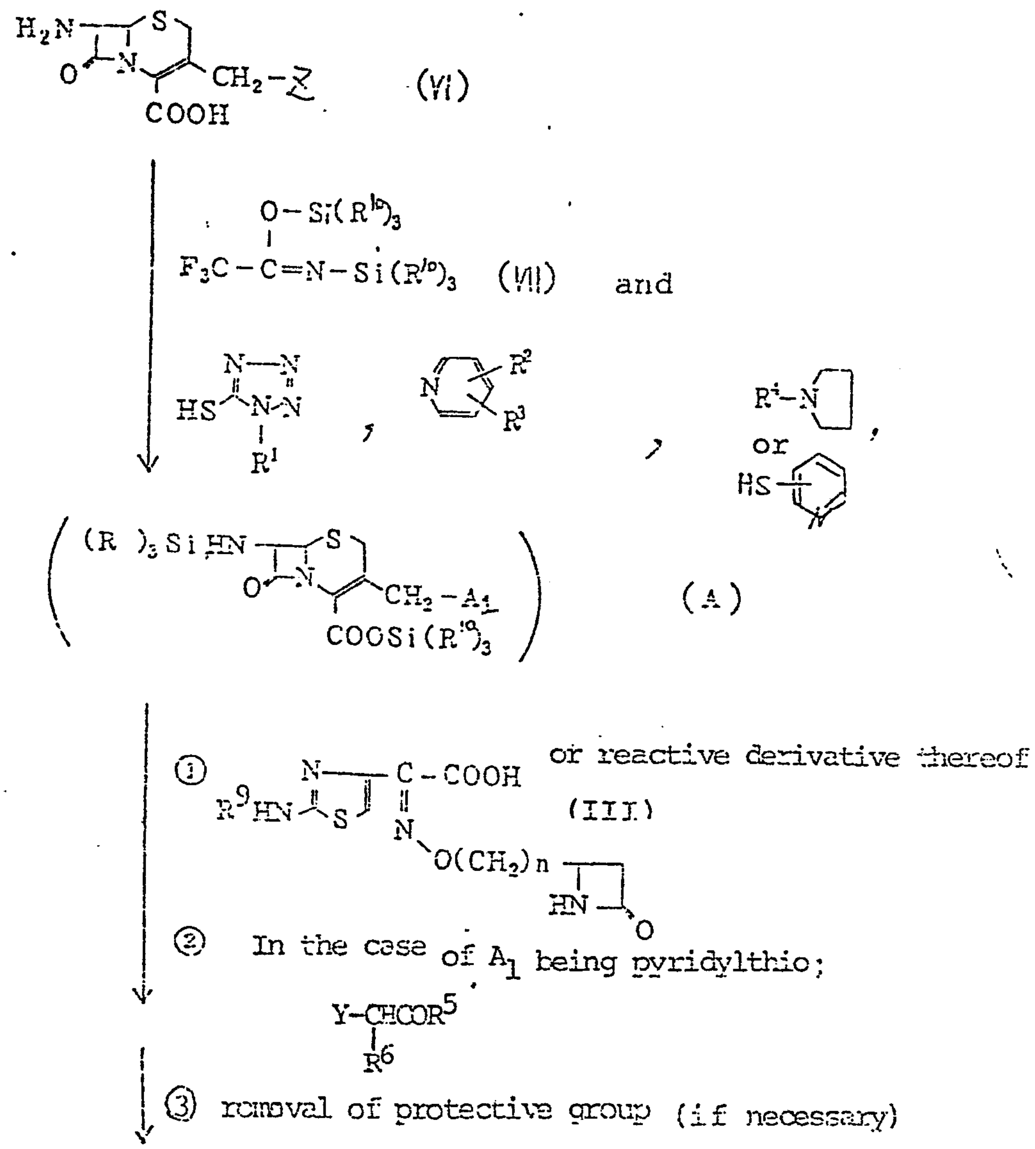


Compound (I)

In the above formulae, Z represents a halogen atom, and $R^{10}$ represents a lower alkyl group. Other symbols are as defined above.

In this process, first, 7-amino-3-halogenomethyl-$\Delta^3$-cephem-4-carboxylic acid (VI) or salt thereof is reacted with N,O-bis(tri-loweralkylsilyl)trifluoro-acetamide (VII) and compound selected from 5-mercapto-1-substituted-tetrazole, substituted pyridine, substituted pyrrolidine, and mercaptopyridine as shown above in an organic solvent which is inert for the reaction. This first reaction can be performed in 1 step or in 2 steps.

The above reaction proceeds easily at room temperature. When performing the reaction in 2 steps, the 1st step may be performed at room temperature and the 2nd step may be performed under cooling. Thus the reaction conditions may be changed for each step. Thus, the compound (A) having protective groups [silyl type groups] is formed.

Then the reaction solution containing compound (A) is reacted with compound of formula (III) or reactive derivative thereof, and, in the case of $A_1$ being pyridylthio, reacted further with halogen compound of formula (V); after removing the protective group(s), the desired compound (I) is obtained.

Process 3:

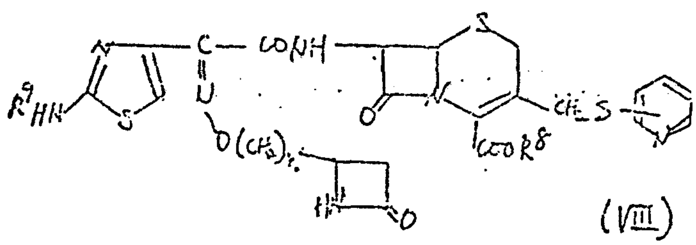

(VIII)

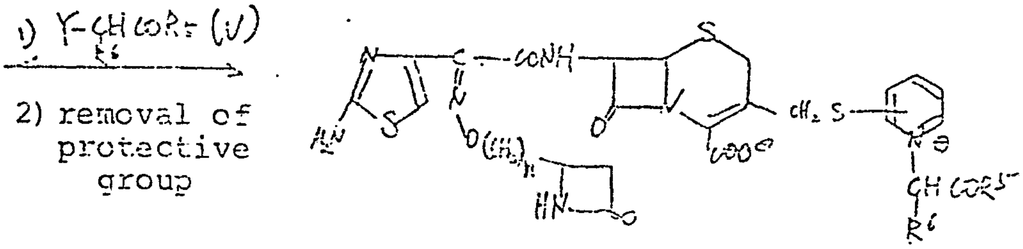


($I_a$)

Compounds ($I_a$) having 1-substituted-pyridiniothio-methyl at the 3-position of the cephem nucleus can be produced by reacting 3-pyridinylthiomethyl-$\Delta^3$-cephem-4-carboxylic acid derivative (VIII) with compound (V), and then, if necessary, removing the protective group(s). The reaction conditions are similar to those for Process 1 ii) in the case of $A_1$ being pyridylthio.

Process 4:

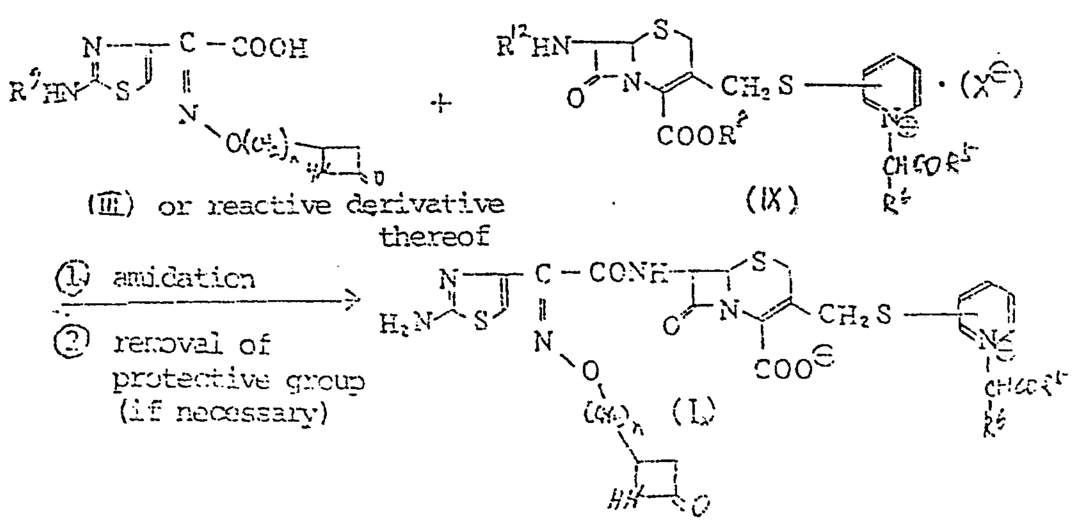

Process 5:

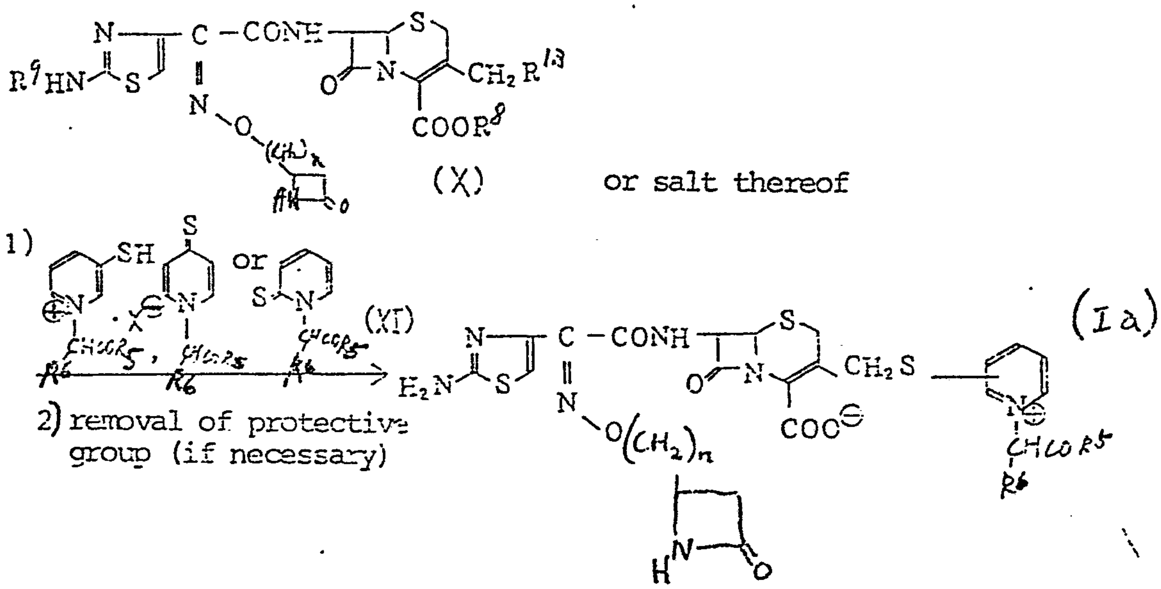


(X) or salt thereof

(XI)

(Ia)

In the above formulae, $R^{12}$ represents a hydrogen atom or a tri(lower)alkylsilyl group;

$-COOR^8$ represents -COOH, -COO-protective group, or $-COO^-$; $(X^-)$ represents an anion when $-COOR^8$ is -COOH or -COO-protective group; however, $X^-$ does not exist if $-COOR^8$ represents $-COO^-$; and $R^{13}$ represents an acyloxy group or a halogen atom.

According to Process 4, compounds ($I_a$) can also be produced by reacting 7-amino- or tri(lower)alkylsilyl-amino-3-(1-substituted-pyridinio)thiomethyl-$\Delta^3$-cephem-4-carboxylic acid derivative of formula (IX) with $\alpha$-(4-oxo-2-azetidinyl-substituted-imino)thiazolylacetic acid of formula (III) or reactive derivative thereof.

Examples of acyloxy in the definition of $R^{13}$ are acetoacetoxy, acetoxy, propionyloxy, iso-propionyloxy, butyryloxy, iso-butyryloxy, pentyloxy, etc.

The reaction is usually performed in a solvent under cooling at room temperature or below. Any solvents which do not take part in the reaction can be used. Examples of the solvent usually used are water and organic solvents such as acetone, dioxane, ether, tetrahydrofuran, ethylmethylketone, chloroform, dichloroethane, ethyl acetate, ethyl formate, dimethylformamide, dimethyl sulfoxide; these solvents may be used alone or in appropriate combination.

According to Process 5, compounds ($I_a$) can be produced by reacting 7-($\alpha$-substituted-thiazolyl-$\alpha$-(4-oxoazetidinyl-substituted-imino)acetamido-3-(acyloxy- or halogenomethyl)-$\Delta^3$-cephem-4-carboxylic acid derivative [X] or salt thereof with 1-substituted-2 or 4-thioxopyridine or 1-substituted-3-mercaptopyridinium salt of formula (XI), and then, if necessary, releasing any protective group(s).

The reaction is usually performed in a solvent. Examples of the solvent are water, phosphoric acid buffer

and organic solvents such as dimethyl sulfoxide, dimethylformamide, acetone, chloroform, methylene chloride, dichloroethane, methanol, ethanol, ether, dioxane, tetrahydrofuran; these sovents may be used alone or in appropriate combination (for example, a mixture of water and . organic solvent). If compound X is used as the free carboxylic acid, for promoting the reaction, a basic compound such as sodium hydroxide, potassium hydroxide, sodium carbonate, etc. may be added to the reaction system. A catalyst such as sodium iodide or potassium iodide may be added for promoting the reaction. The reaction proceeds easily at room temperature or under heating.

A salt of the formula I compound can be produced by performing a foregoing production process using a salt of the starting compound, or by applying a salt-forming reaction to the free formula I compound.

The formula I compounds and salts can be separated and purified in conventional manner, such as extraction with organic solvent, crystallization, or column chromatography.

The production processes of this invention are further described in the following Examples. In addition, the raw materials used in the process of this invention include novel compounds and examples of the production of these compounds are also shown in the Reference Examples.

Reference Example 1.

(a)

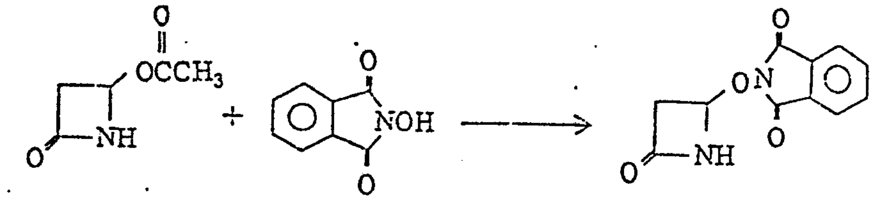

To 200 ml of water was added 12.9 g of 4-acetoxy-2-azetidine and the mixture was cooled to 0°C. 100 ml of cooled solution of 16.3 g of N-hydroxyphthalimide in 1N aqueous sodium hydroxide wad added to the mixture over a period of 30 minutes at 0 to 5°C. After stirring the mixture for 30 minutes at the same temperature and then for a further 4 hours at room temperature, the crystals formed were collected by filtration. The crystals thus obtained were washed with water, and dried over phosphorus pentoxide under reduced pressure to give 16.9 g of 2-azetidinone-4-oxyphthalimide.

(i) NMR (DMSO-$d^6$) $\delta$ ppm

2.5 ~ 3.5 (2H, m, 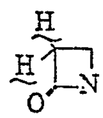 )

5.75 (1H, d, 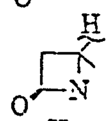 )

7.92 (4H, s, 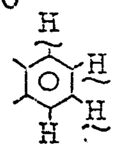 )

8.91 (1H, broad s, 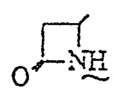 )

(ii) ($C_{11}H_8N_2O_4$)

| C(%) | H(%) | N(%) |
|---|---|---|
| 56.90 | 3.47 | 12.06 |
| 56.17 | 3.30 | 11.98 |

(b)

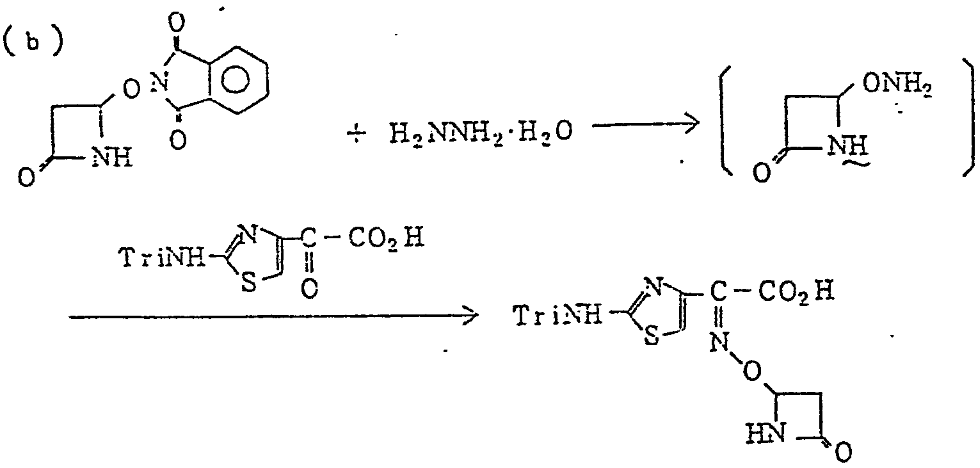


In 90 ml of dichloromethane was suspended 3 g of 2-azetidinone-4-yloxyphthalimide, and after adding thereto 628 1 of hydrazine hydrate, the mixture was stirred for 30 minutes at room temperature. Insoluble materials were removed by filtration to give a pale yellow clear solution of O-2-azetidinone-4-ylhydroxylamine. A solution consisting of 3.85 g of 2-(2-tritylamino-4-thiazolyl)glyoxylic acid, 56 ml of dichloromethane, and 19 ml of methanol was cooled to 15°C. To this solution was added the pale yellow solution obtained above, and the mixture thus obtained was stirred for 2 hours at room temperature. The reaction solution was concentrated under reduced pressure, and after adding ether to the residue, crystals formed were collected by filtration, washed with ether, and dried under reduced pressure to give 4.31 g of (Z)-α-(4-oxo-2-azetidinyloxyimino)-α-(2-tritylamino-4-thiazolyl)acetic acid.

(i) NMR ($d_6$-DMSO $-D_2O$)

2.7 ~ 3.3 (2H, m, 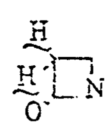 )

5.83 (1H, q, 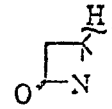 )

6.93 (1H, s, 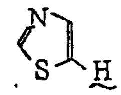 )

8.80 (1H, Broad s, Tri-NH-)

Reference Example 2

(a)

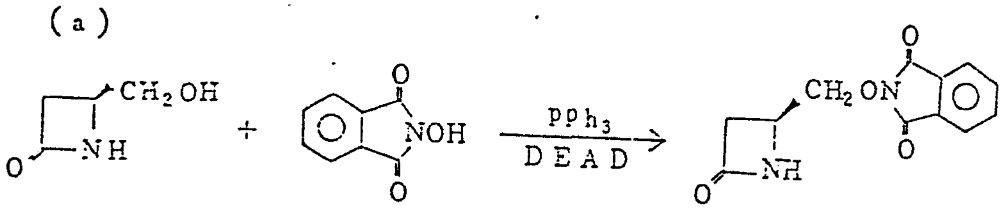


In dry tetrahydrofuran were dissolved 1.05 g of (S)-4--hydroxymethyl-2-oxoazetidine and 1.63 g of N-hydroxyphthalimide and after adding thereto 2.62 g of triphenylphosphine, the mixture was cooled with ice-water. After adding thereto dropwise 1.74 g of azodicarboxylic acid diethyl ester, the mixture was stirred for 1 hour at room temperature. Insoluble materials were removed by filtration, and the filtrate was concentrated under reduced pressure. The residue was subjected to silica gel column chromatography. The product was eluted with chloroform-ethyl acetate (9:1) to provide 1.11 g of (S)-4-oxoazetidinyl-2-methoxyphthalimide.

(i) NMR ($CDCl_3$) δ ppm

2.4 ~ 3.3 (2H, m, 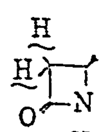 )

4.1 (1H, m, 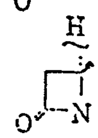 )

4.32 (2H, q, 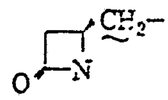 )

6.7 (1H, Broad s, 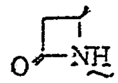 )

7.84 (4H, s, 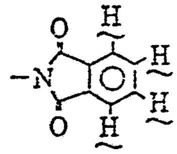 )

(ii) IR (KBr) $cm^{-1}$

3360, 3250, 1770, 1750, 1720

(b)

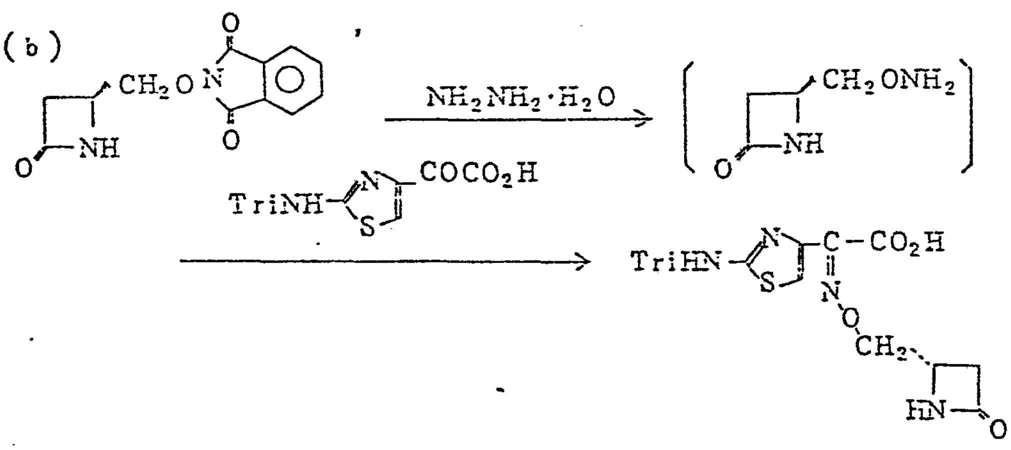


In 15 ml of dichloromethane was dissolved 1.11 g of (S)-4-oxoazetidinyl-2-methoxyphthalimide and after adding thereto 0.22 ml of hydrazine hydrate, the mixture was stirred for 30 minutes at room temperature. Insoluble materials were removed by filtration to give a solution containing O-(4-oxo-2-azetidinyl)methylhydroxylamine.

To a solution of 1.6 g of 2-(2-tritylamino-4-thiazolyl)glyoxylic acid in a mixture of 15 ml of methanol and dichloromethane was added the above hydroxylamine solution and after stirring it for 1 hour, solvents

were distilled away under reduced pressure and the residue was treated with ether to give 1.92 g of (Z)-α-(4-oxo-2-azetidinylmethoxyimino)-α-(2-tritylamino-4-thiazolyl)-acetic acid.

(i) NMR ($d_6$ − DMSO) δ ppm

2.4 ∼ 3.1 (2H, m, 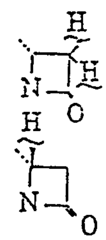 )

3.7 (1H, m, )

4.12 (2H, d, 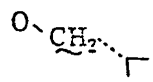 )

6.91 (1H, s, 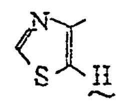 )

(ii) IR (KBr) $cm^{-1}$

1770, 1730

(iii) FABMS $[M+H]^+$ 513

Example 1

(a)

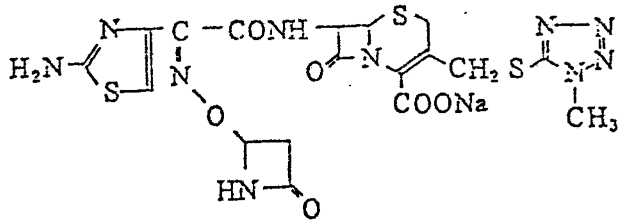

In 10 ml of dichloromethane was dissolved 498 mg of (Z)-α-(4-oxo-2-azetidinyloxyimino)-α-(2-tritylamino-4-thiazolyl)acetic acid and after cooling the solution to -10°C and adding thereto 208 mg of phosphorus pentachloride, the mixture was stirred for 30 minutes at -13° to -3°C to provide Solution A.

494 mg of 7-amino-3-(1-methyl-5-tetrazolyl)thiomethyl-$\Delta^3$-cephem-4-carboxylic acid diphenylmethyl ester was dissolved in 10 ml of dichloromethane and after cooling the solution to -40°C, 560 1 of pyridine was added thereto to provide Solution B.

To Solution B was added dropwise Solution A, and the mixture was stirred for 1 hour at -25° to -20°C. The reaction solution was poured into 30 ml of ice-water and after adjusting the pH of the mixture to 1-2, the organic layer was separated, washed, dried over anhydrous magnesium sulfate, and concentrated. The residue was subjected to silica gel column chromatography, and the product was eluted with benzene-ethyl acetate (5:1) to

provide 518 mg of (Z)-7-[α-(4-oxo-2-azetidinyloxyimino)-α-(2-tritylamino-4-thiazolyl)acetamido]-3-(1-methyl-5-tetrazolyl)thiomethyl-$\Delta^3$-cephem-4-carboxylic acid benzhydryl ester.

(i) NMR ($d^6$-DMSO) δ ppm

2.8～3.3 (2H, m, 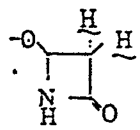 )

3.5～4.0 (2H, 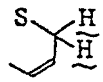 )

4.1～4.5 (2H, 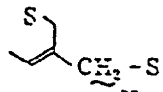 )

5.18 (1H, d, 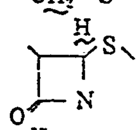 )

5.6 (1H, m, 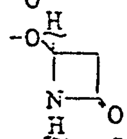 )

5.75 (1H, dd, 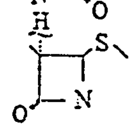 )

6.82, 6.83 (1H, 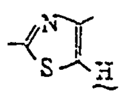 )

6.88 (1H, s, 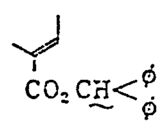 )

(ii) IR (KBr) $cm^{-1}$

1775, 1715, 1675, 1520

(iii) FABMS $[M+H]^+$ 975

(b) 500 mg of (Z)-7-[α-(4-oxo-2-azetidinyloxyimino)-α-(2-tritylamino-4-thiazolyl)acetamido]-3-(1-methyl-5-tetrazolyl)thiomethyl-$\Delta^3$-cephem-4-carboxylic acid benzhydryl ester was added to a cooled mixture of 3.5 ml of trifluoroacetic acid and 0.4 ml of anisole and after stirring the reaction mixture for 1.5 hour at room temperature, the reaction solution was concentrated under reduced pressure. Ether was added to the residue to form powder, and the powder was collected by filtration. The collected powder was dissolved in 3 ml of trifluoroacetic acid and after adding thereto 1 ml of water, the mixture was stirred for 1.5 hour at room temperature. The reaction solution was concentrated under reduced pressure and after adding thereto ether, triturating, and then filtering, 335 mg of solid material was obtained. 250 mg of the solid material (a portion of the solid material obtained) was dissolved in aqueous sodium hydrogen carbonate, and the solution formed was subjected to column chromatography on Diaion HP-20. The product was eluted with water and methanol in a mixing ratio changing successively from pure water to 20 % methanol-water. Fractions containing the desired product were collected, and concentrated to provide 57 mg of sodium (Z)-7-[α-(4-oxo-2-azetidinyloxyimino)-α-(2-amino-4-thiazolyl)acetamido]-3-(1-methyl-5-tetrazolyl)thiomethyl-$\Delta^3$-cephem-4-carboxylate.

(i) FABMS $[M+H]^+$ 589

(ii) IR (KBr) $cm^{-1}$

1755, 1660, 1600

(iii) NMR ( $d^6$ − DMSO ) δ ppm

| | | | |
|---|---|---|---|
| 3.5～3.9 | ( 2 H, | 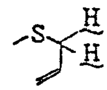 | ) |
| 3.92 | ( 3 H, | 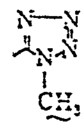 | ) |
| 4.12～4.48 | ( 2 H, | 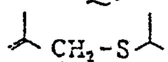 | ) |
| 5.14 | ( 1 H, | 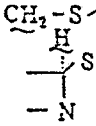 | ) |
| 5.64 | ( 1 H, | 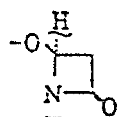 | ) |
| 5.76 | ( 1 H, | 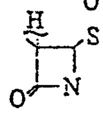 | ) |
| 6.76, 6.80 | ( 1 H, | 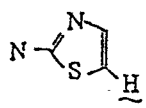 | ) |

Example 2

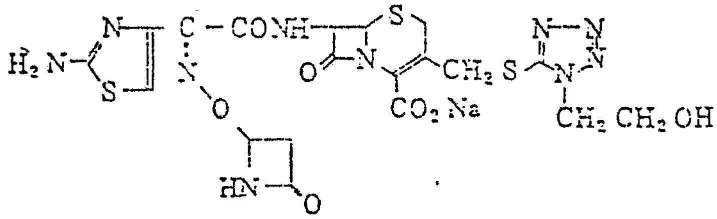

(a) 498 mg of (Z)-α-(4-oxo-2-azetidinyloxyimino)-α-(2-tritylamino-4-thiazolyl)acetic acid was dissolved in 20 ml of dichloromethane and after adding thereto 208 mg of phosphorus pentachloride at 3°C, the mixture was stirred for 15 minutes to provide Solution A.

358 mg of 7-amino-3-[1-(2-hydroxyethyl)-5-tetrazolyl]thiomethyl-$\Delta^3$-cephem-4-carboxylic acid was suspended in 3 ml of N,N-dimethylacetamide and after adding thereto 0.8 ml of N,O-bistrimethylsilyltrifluoroacetamide, the mixture was stirred for 30 minutes at room temperature to give a clear solution. The solution was cooled to -40°C and after adding thereto 10 ml of dichloromethane, 0.4 ml of pyridine was further added to provide Solution B.

To Solution B was added Solution A, and the mixture was stirred for 15 minutes at -20°C. To the reaction solution was added 5.4 ml of 0.5 N hydrochloric acid and after stirring for 10 minutes, the mixture was concentrated under reduced pressure. To the residue was added 30 ml of water, and the mixture was filtered to give solid material.

(b) 10 ml of trifluoroacetic acid was cooled to 0°C and after adding thereto the solid material obtained above to provide a clear solution, and adding further 1 ml of water, the solution was stirred for 1 hour at room temperature. The reaction solution was concentrated under reduced pressure, and ether was added to the residue. Solid material was collected by filtration, and dissolved in aqueous sodium hydrogen carbonate. The solution was subjected to column chromatography on MCI GEL(CHP20P), and the product was eluted with 6-10 % methanol-water. Fractions obtained were concentrated and lyophilized to provide 75 mg of sodium (Z)-7-[α-(2-amino-4-thiazolyl)-α-(4-oxo-2-azetidinyloxyimino)acetamido]-3-[1-(2-hydroxyethyl)-5-tetrazolyl]thiomethyl-$\Delta^3$-cephem-4-carboxylate.

(i) NMR ($d_6$-DMSO) δ ppm

2.77~3.28 (2H, m, 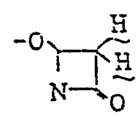 )

3.74 (2H, m, 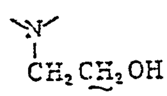 )

4.1~4.4 (4H, m, 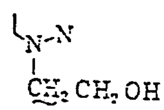 と 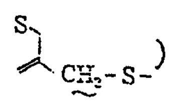 )

4.99 (1H, d, 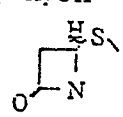 )

5.5~5.7 (2H, m, 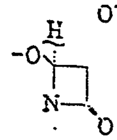 と 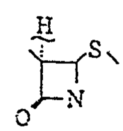 )

6.79 と 6.81 (1H, s, 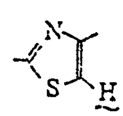 )

(ii) IR (KBr) $cm^{-1}$

1770 , 1755, 1660, 1600

(iii) FABMS [M+H]$^+$ 619

Example 3

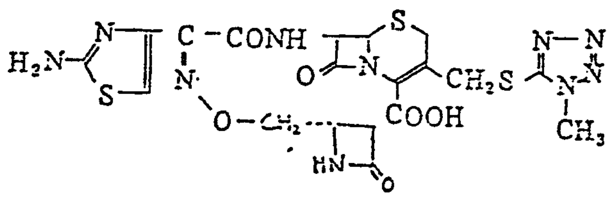

(a) In 20 ml of dichloromethane was dissolved 512 mg of (Z)-α-(4-oxo-2-azetidinylmethoxyimino)-α-(2-tritylamino-4-thiazolyl)acetic acid and after cooling the solution to -3°C and adding thereto 208 mg of pentachloride, the mixture was stirred for 10 minutes to provide Solution A.

494 mg of7-amino-3-(1-methyl-5-tetrazolyl)thiomethyl-$\Delta^3$-cephem-4-carboxylic acid diphenylmethyl ester was dissolved in 20 ml of dichloromethane and after cooling the solution to -40°C, 564 μl of pyridine was added thereto to provide Solution B.

To Solution B was added Solution A, and the mixture was stirred for 1 hour at -20°C. To the reaction solution were added 50 ml of dichloromethane and 20 ml of ethyl acetate and after adding thereto 50 ml of ice-water, the mixture was stirred vigorously for 15 minutes. The organic layer was separated, washed with ice-water, and dried over anhydrous magnesium sulfate. Solvent was distilled away under reduced pressure. The residue was subjected to silica gel column chromatography and the product was eluted with chloroform-ethyl acetate (3:1) to provide 398 mg of (Z)-7-[α-(4-oxo-2-azetidinylmethoxyimino)-α-(2-tritylamino-4-thiazolyl)acetamido]-3-(1-methyl-5-

tetrazolyl)thiomethyl-$\Delta^3$-cephem-4-carboxylic acid benzhydryl ester.

(i) NMR ( $CDCl_3$ ) $\delta$ ppm

2.48~3.18 ( 2H, m, 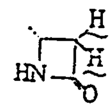 )

3.71 ( 2H, s, 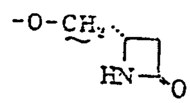 )

3.81 ( 3H, s, 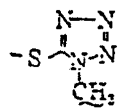 )

5.02 ( 1H, d, 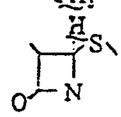 )

5.97 ( 1H, dd, 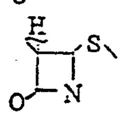 )

6.68 ( 1H, s, 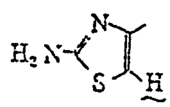 )

6.92 ( 1H, s, $-CH<^{\phi}_{\phi}$ )

(ii) IR (KBr) $cm^{-1}$

1765, 1730, 1670, 1520

(iii) FABMS $[M+H]^+$ 989

(b) 320 mg of (Z)-7-[α-(4-oxo-2-azetidinylmethoxyimino)-α-(2-tritylamino-4-thiazolyl)acetamido]-3-(1-methyl-5-tetrazolyl)thiomethyl-$\Delta^3$-cephem-4-carboxylic acid benzhydryl ester was added to a cooled mixture (0°C) of

2.5 ml of trifluoroacetic acid and 0.4 ml of anisole, and the mixture was stirred for 1 hour at room temperature. The reaction solution was concentrated under reduced pressure, and ether was added thereto to form powder. The powder was collected by filtration, and dissolved in 3 ml of trifluoroacetic acid. 1 ml of water was added to the solution and after concentrating the reaction solution under reduced pressure, adding ether to the residue, and then triturating, 170 mg of (Z)-7-[α-(4-oxo-2-azetidinylmethoxyimino)-α-(2-amino-4-thiazolyl)-acetamido]-3-(1-methyl-5-tetrazolyl)thiomethyl-$\Delta^3$-cephem-4-carboxylic acid was collected by filtration.

(i) NMR ($d_6$ − DMSO) δ ppm

| | | | |
|---|---|---|---|
| 2.4～3.1 | (2H, m, | 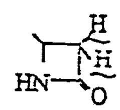 | ) |
| 3.71 | (2H, s, | 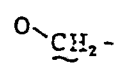 | ) |
| 3.92 | (3H, s, | 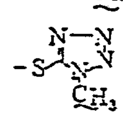 | ) |
| 5.14 | (1H, d, | 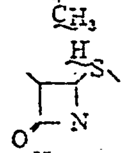 | ) |
| 5.80 | (1H, q, | 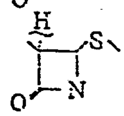 | ) |
| 6.83 | (1H, s, | 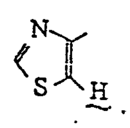 | ) |

(ii) IR (KBr) $cm^{-1}$

1775, 1730, 1670

(iii) FABMS $[M+H]^+$ 581

Example 4 

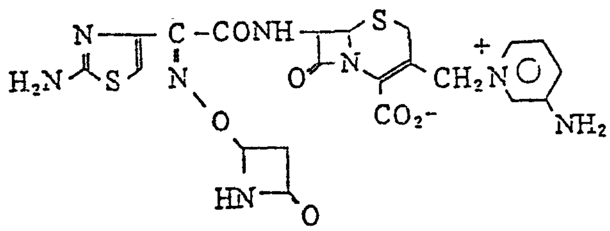

(a) In 20 ml of dichloromethane was suspended 680 mg of 7-amino-3-iodomethyl- 3-cephem-4-carboxylic acid and after adding thereto 1.14 ml of N,O-bis(trimethylsilyl)-trifluoroacetamide, the mixture was stirred for 30 minutes at room temperature to give a clear solution. To the solution was added 188 mg of 3-aminopyridine, and the mixture was stirred for 4 hours at room temperature to provide a solution containing trimethylsilyl 7-trimethylsilylamino-3-(3-aminopyridiniomethyl)-$\Delta^3$-cephem-4-carboxylate iodide.

996 mg of (Z)-α-(4-oxo-2-azetidinyloxyimino)-α-(2-tritylamino-4-thiazolyl)acetic acid (obtained in Reference Ex. 1) was dissolved in 30 ml of dichloromethane and after cooling the solution to -10°C and adding thereto 416 mg of phosphorus pentachloride, the mixture was stirred for 15 minutes at 0°C to provide Solution A.

A solution containing trimethylsilyl 7-trimethylsilylamino-3-(3-aminopyridiniomethyl)-$\Delta^3$-cephem-4-carboxylate iodide (obtained at 4 (a)] was cooled to -50°C, and 0.8 ml of pyridine was added to the solution.

To the formed solution was added Solution A and the temperature of the mixture was increased to -15°C over a period of 30minutes. To the reaction solution were added 5.4 ml of 1N-hydrochloric acid, 6.7 ml of water and 6.7 ml of tetrahydrofuran, and the mixture was stirred for 10 minutes at 0°C. The reaction solution was concentrated under reduced pressure and after adding 66 ml of water to the residue, precipitates formed were collected by filtration.

(b) 20 ml of trifluoroacetic acid was cooled to 5°C and after adding thereto the precipitates (obtained at 4 (a)) and then adding 1 ml of water, the mixture was stirred for 1 hour at room temperature. The reaction solution was concentrated under reduced pressure and after adding to the residue 2 ml of ethanol and then adding 40 ml of ether to form powder, the formed powder was collected by filtration to give 910 mg of a crude product. To the crude product thus obtained was added 50 ml of water and 5 ml of 1N-hydrochloric acid to dissolve it, and insoluble material (remaining in a small amount] was removed by filtration. The filtrate was subjected to column chromatography on Diaion HP-20, and the product was eluted with 13-20 % methanol-water. Fractions containing the desired product were collected, concentrated, and lyophilized to give 160 mg of (Z)-7-[α-(2-amino-4-thiazolyl)-α-(4-oxo-2-azetidinyloxyimino)acetamido]-3-(3-aminopyridiniomethyl)-$\Delta^3$-cephem-4-carboxylate.

(i) NMR ( $d_6$ − DMSO ) δ ppm

| | | |
|---|---|---|
| 2.8～3.2 | ( 2 H, m, | 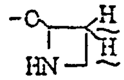 ) |
| 3.2 5 | ( 2 H, q, | 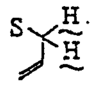 ) |
| 4.9 2～5.7 | ( 2 H, q, | 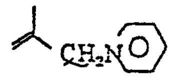 ) |
| 5.0 7 | ( 1 H, d, | 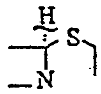 ) |
| 5.6 1 | ( 1 H, d d, | 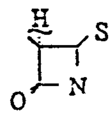 ) |
| 6.7 9, 6.6 8 | ( 1 H, | 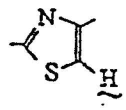 ) |
| 7.6 2 | ( 1 H, s, | 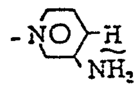 ) |
| 7.6 5 | ( 1 H, d, | 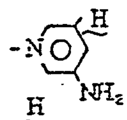 ) |
| 8.4 2 | ( 1 H, d, | 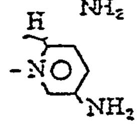 ) |
| 8.5 4 | ( 1 H, s, | 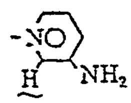 ) |

(ii) I R ( K B r ) $cm^{-1}$

1755 ( Broad ), 1660～1600

(iii) FABMS $[M+H]^+$ 546

0176369

Example 5

(a)

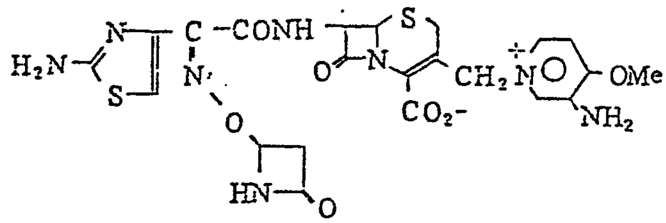

Following the same procedure as in Example 4 using 3-amino-4-methoxypyridine instead of 3-aminopyridine, (Z)-7-[α-(2-amino-4-thiazolyl)-α-(4-oxo-2-azetidinyloxyimino)acetamido]-3-(3-amino-4-methoxy-pyridiniomethyl)-$\Delta^3$-cephem-4-carboxylate was obtained.

(i) NMR ( $d_6$ − DMSO ) δ ppm

2.7～3.2 ( 2 H, m, 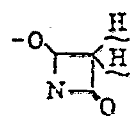 )

3.4 ( 2 H, q, 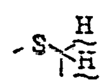 )

4.04 (3H, s, -OMe )

5.16 (2H, q, 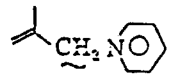 )

5.06 (1H, d, 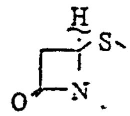 )

5.6 (1H, m, 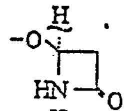 )

5.6 (1H, q, 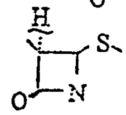 )

6.83, 6.85 (1H, s, 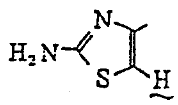 )

7.43 (1H, d, 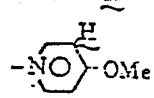 )

8.42 (1H, s, 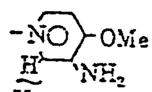 )

8.50 (1H, d, 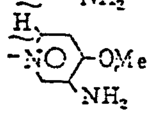 )

(ii) IR (KBr) $cm^{-1}$

1770, 1755, 1660, 1610

(iii) FABMS $[M+H]^+$ 575

Example 6

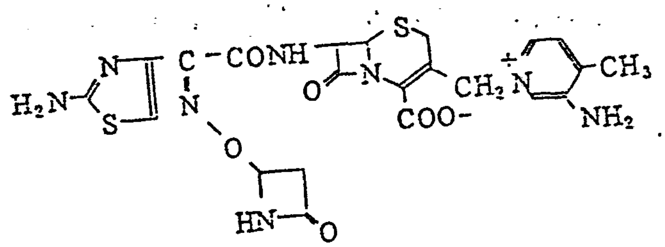

Following the same procedure as in Example 4 using 3-amino-4-methylpyridine instead of 3-aminopyridine, (Z)-7-[α-(2-amino-4-thiazolyl)-α-(4-oxo-2-azetidinyloxyimino)acetamido]-3-(3-amino-4-methylpyridiniomethyl)-$\Delta^3$-cephem-4-carboxylate was obtained.

(i) NMR ( $d_6$ — DMSO ) δ ppm

2.26 ( 3H, s, 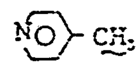 )

2.6~3.6 ( 4H. m, 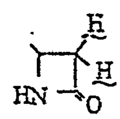 , 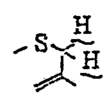 )

5.06 ( 1H, d, 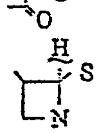 )

5.60 ( 1H, d, 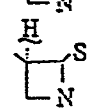 )

{ 6.78
  6.79 ( 1H, 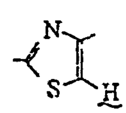 )

7.56 (1H, d, 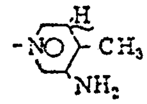 )

8.26 (1H, d, 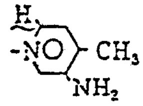 )

8.37 (1H, s, 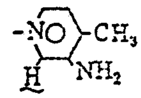 )

(ii) IR (KBr) $cm^{-1}$

1770 , 1755, 1660~1600, 1525

(iii) FABMS $[M+H]^+$ 559

Example 7

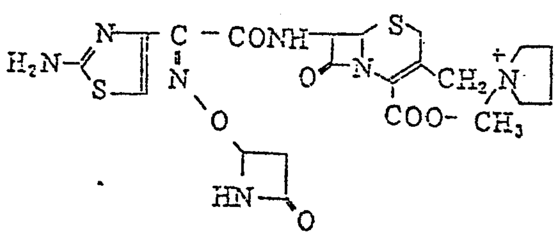

By following the same procedure as in Example 4 using 1-methylpyrrol instead of 3-aminopyridine, (Z)-7-[ -(2-amino-4-thiazolyl)- -(4-oxo-2-azetidinyloxyimino)-acetamido]-3-(1-methyl-1-pyrrolidiniomethyl)-3-cephem-4-carboxylate was obtained.

(i) IR (KBr) $cm^{-1}$

1770 , 1755, 1660~1610, 1525

(ii) FABMS $[M+H]^+$ 536

Example 8

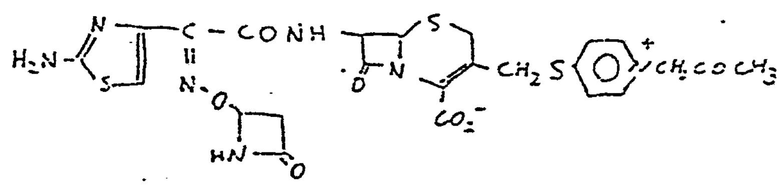

a) in 18 ml of water was dissolved 771 mg of sodium hydrogen carbonate, and 1.78 g of 7-aminocephalosporanic acid was added portionwise thereto and dissolved by heating at 38°C. After adding thereto 800 mg of 4-mercaptopyridine, the mixture was allowed to react for 4 hours while stirring at 55-60°C. To the reaction solution was added 75 1 of acetic acid and after cooling the mixture, crystals were collected by filtration, washed with water, and dried to give 1.33 g of 7-amino-3-(4-pyridylthio)methyl-$\Delta^3$-cephem-4-carboxylic acid.

b) In 10 ml of dichloromethane was suspended 900 mg of 7-amino-3-(4-pyridylthio)methyl-$\Delta^3$-cephem-4-carboxylic acid (obtained at 8 (a)] and after adding thereto 2.6 ml of N,O-bis(trimetlhylsilyl)trifluoroacetamide, the mixture was stirred for 1.5 hour at room temperature to dissolve it. The solution was cooled to -50°C, and 880 $\mu$l of pyridine was added thereto to provide Solution A.

1.38 g of (Z)-α-(4-oxo-2-azetidinyloxyimino)-α-(2-tritylamino-4-thiazolyl)acetic acid was dissolved in 20 ml of dichloromethane and after adding thereto 576 mg of phosphorus pentachloride, the mixture was stirred for 10 minutes to provide Solution B.

Solution B was added Solution A, and the mixture was allowed to react for 1 hour at -20°C. After adding thereto 7.5 ml of N-hydrochloric acid and 30 ml of water, the mixture was stirred for 30 minutes at 0°C. The reaction mixture was concentrated and after adding thereto water, precipitates were collected by filtration to give 1.91 g of /a crude product of (Z)-7-[α-(4-oxo-2-azetidinyloxyimino)-α-(2-tritylamino-4-thiazolyl)acetamido]-3-(4-pyridylthio)-methyl-$\Delta^3$-cephem-4-carboxylic acid.

c) 1.91 g of the crude product (obtained at 8 (b)) was dissolved in 30 ml of dichloromethane and after cooling the solution to 15°C, a solution of diphenyl-diazomethane was added thereto. Solvent was distilled away under reduced pressure, and ether was added to form powder. The powder was collected by filtration and subjected

to silica gel column chromatography. The product was eluted with chloroform-ethyl acetate (1:1). Fractions containing the desired product were collected, and concentrated to give 0.41 g of (Z)-7-[α-(4-oxo-2-azetidinyloxyimino)-α-(2-tritylamino-4-thiazolyl)acetamido]-3-(4-pyridylthio)methyl-$\Delta^3$-cephem-4-carboxylic acid benzhydryl ester.

IR (KBr) $cm^{-1}$: 1770, 1755, 1725, 1680

FABMS $[M+H]^+$: 970

d) In 1 ml of dimethylsulfoxide was dissolved 0.4 g of (Z)-7-[α-(4-oxo-2-azetidinyloxyimino)-α-(2-tritylamino-4-thiazolylacetamido]-3-(4-pyridylthio)methyl-$\Delta^3$-cephem-4-carboxylic acid benzhydryl ester and after adding thereto 117 ml of chloroacetone, the mixture was stirred overnight. The solution was added to a cooled solution (10°C) consisting of 23 ml of trifluoroacetic acid and 0.2 ml of anisole, and the mixture was allowed to react for 30 minutes at 20°C. The reaction solution was cooled to 10°C and after adding thereto 5 ml of water, the mixture was stirred for 1 hour at 20°C. Solvent was distilled away and after adding ether to the residue, residual oily material was obtained by decantation. The oily material was subjected to column chromatography on Diaion HP-20, and the product was eluted with 30 % methanol-water. Fractions containing the desired product were collected, and concentrated. The material obtained by concentration was subjected to column chromatography on Diaion HP-20 to remove the undesired product, and 30 mg of (Z)-1-acetonyl-4-[[[7-[2-(2-amino-4-thiazolyl)-2-(4-oxo-2-

azetidinyloxyimino)acetamido] -4-carboxy-$\Delta^3$-cephem-3-yl] -methyl] thio] pyridinium was obtained.

i) NMR ($d_6$-DMSO) δ ppm

2.26 (3H, s, -COC$\underline{H}_3$)

5.02 (1H, d, 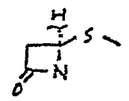 )

5.54 (4H, m, 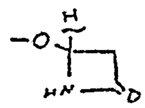 and 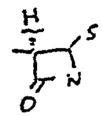 and 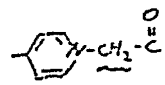 )

6.80 (1H, 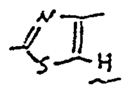 )

7.24 (2H, 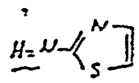 )

8.40 (4H, s, 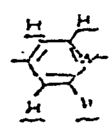 )

8.78 (1H, 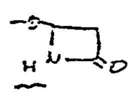 )

9.56 (1H, d, -CON$\underline{H}$-)

ii) IR (KBr) $cm^{-1}$

1765

iii) FABMS (M+H) 618

Claims:

1. A cephalosporin compound of the following general formula (I), or a pharmacologically acceptable salt thereof

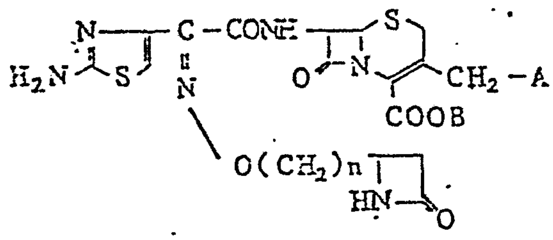

(I)

wherein A represents

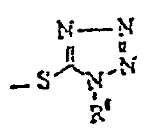 , 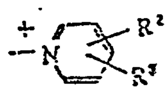 , 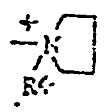 , or 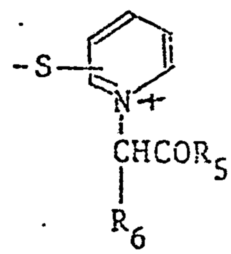

wherein $R^1$ represents a hydrogen atom or a $C_1$ to $C_5$ alkyl group which is unsubstituted or substituted by at least one hydroxyl group; $R^2$ and $R^3$ are the same or different and selected from an amino group, a hydrogen atom, $C_1$ to $C_5$ alkyl groups and $C_1$ to $C_5$ alkoxy groups; $R^4$ represents a $C_1$ to $C_5$ alkyl group; and $R^5$ and $R^6$ are the same or different and selected from a hydrogen atom and $C_1$ to $C_5$ alkyl groups; -COOB represents -COOH or $-COO^-$; and n represents 0, 1 or 2.

2. A compound according to claim 1 wherein A represents

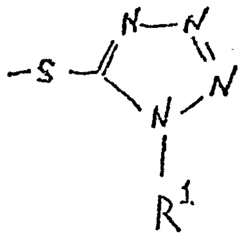

or

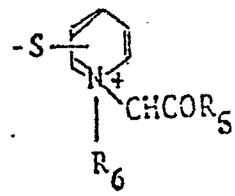

3. A compound according to claim 1 which is 7-[α-(4-oxo-2-azetidinyloxyimino)-α-(2-amino-4-thiazolyl)acetamido]-3-(1-methyl-5-tetrazolyl)thiomethyl-$\Delta^3$-cephem-4-carboxylic acid or a pharmacologically acceptable salt thereof,

7-[α-(2-amino-4-thiazolyl)-α-(4-oxo-2-azetidinyloxyimino)acetamido]-3-[1-(2-hydroxyethyl)-5-tetrazolyl]thiomethyl-$\Delta^3$-cephem-4-carboxylic acid or a pharmacologically acceptable salt thereof,

or

7-[α-(2-amino-4-thiazolyl)-α-(4-oxo-2-azetidinyloxyimino)acetamido]-3-(1-acetonyl-4-pyridiniomethyl)-$\Delta^3$-cephem-4-carboxylate or a pharmacologically acceptable salt thereof.

4. A method of preparing a compound of formula I :

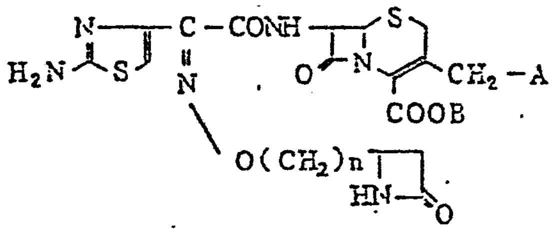

(I)

which comprises reacting substituted oxyiminothiazolylacetic acid derivative of formula (III) or reactive derivative thereof with 7-amino-3-cephem derivative of formula (II) [and, then, in the case of $A_1$ being a pyridylthio group, reacting the formed compound (IV) with compound (V)] and then, if necessary, releasing any protective group(s):

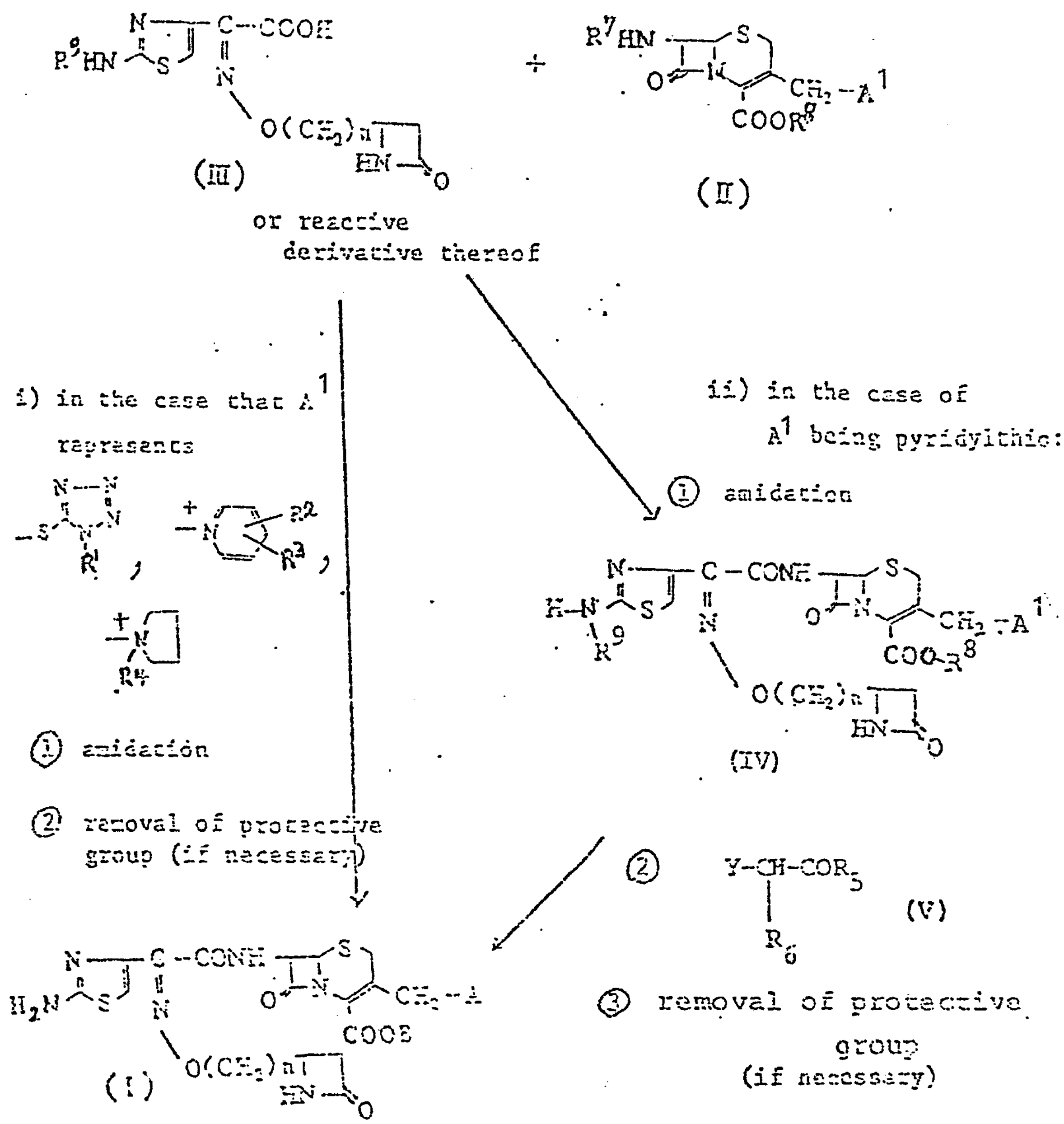


(III) (II) (IV) (V) (I)

wherein $R^1$, $R^2$, $R^3$, $R^4$, $R^5$, $R^6$, -COOB and n are as defined in claim 1, $A^1$ represents

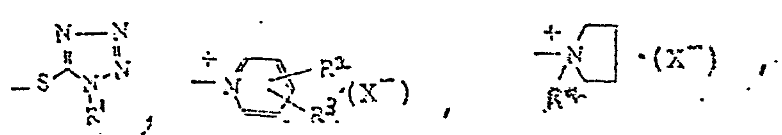

or a pyridylthio group, $R^7$ represents a hydrogen atom or a protective group for an amino group, $R^8$

represents a hydrogen atom or a protective group for a carboxyl group, and $R^9$ represents a hydrogen atom or a protective group for an amino group, Y represents halogen and x represents an anion.

5. A method of preparing a compound of formula I

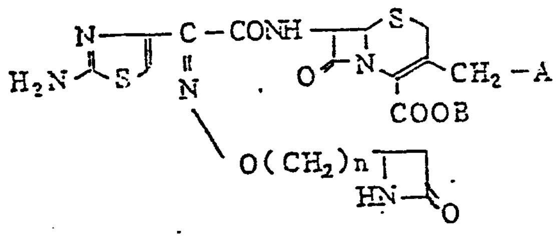

(I)

which comprises reacting 7-amino-3-halogenomethyl-3-cephem-4-carboxylic acid (VI) or salt thereof

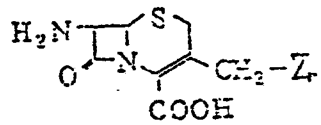

(VI)

with N,O-bis(tri-loweralkylsilyl)trifluoroacetamide (VII)

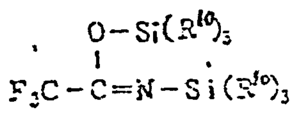

(VII)

and compound selected from 5-mercapto-1-substituted-tetrazole, substituted pyridine, substituted pyrrolidine,

or mercaptopyridine as shown below

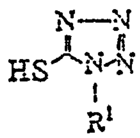 , 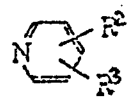 , 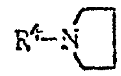 ,

or

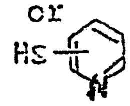

and then reacting the reaction solution containing compound of the formula (A)

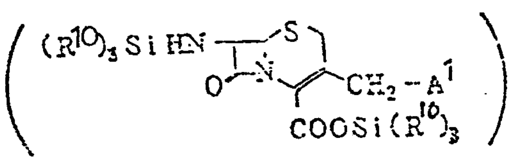

(A)

with compound of formula (III) or reactive derivative thereof

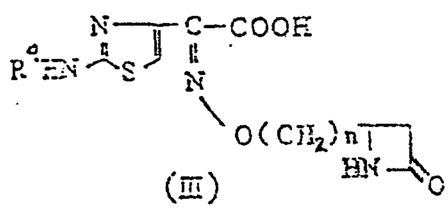

(III) ,

[and, in the case of $A^1$ being pyridylthio, reacting further with halogen compound of formula (V)]

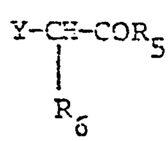

(V) ,

and removing any protective group(s), wherein Z represents

halogen, $R^{10}$ represents a $C_1$ to $C_5$ alkyl group, and the other symbols are as defined in claim 4.

6, A method of preparing a compound of the following formula ($I_a$) :

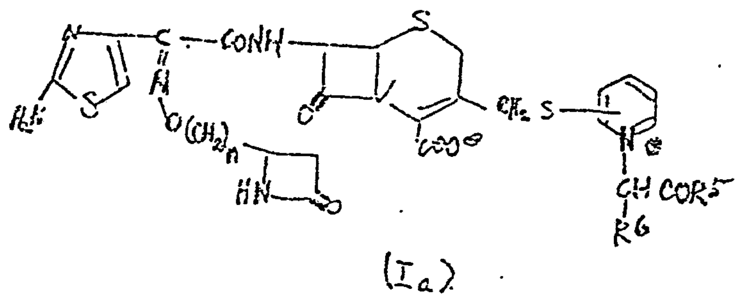

($I_a$)

which comprises :

(i) reacting 3-pyridinylthiomethyl-3-cephem-4-carboxylic acid derivative (VIII)

with compound (V), and then removing any protective group(s),

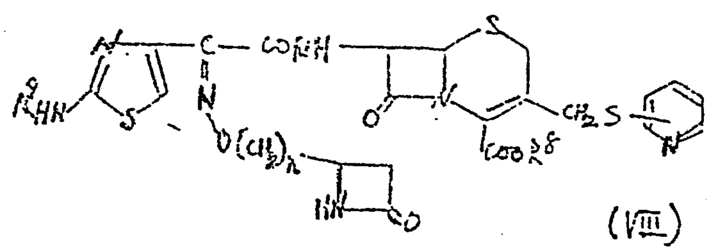

(VIII)

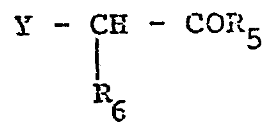

(V)

the symbols being as defined in claim 4,

0176369

or (2) reacting 7-amino- or tri(loweralkylsilyl-amino-3-(1-substituted-pyridinio)thiomethyl-$\Delta^3$-cephem-4-carboxylic acid derivative of formula (IX)

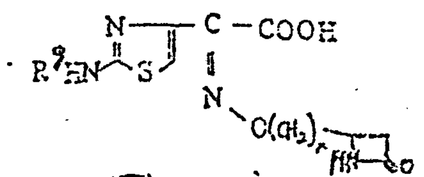

+

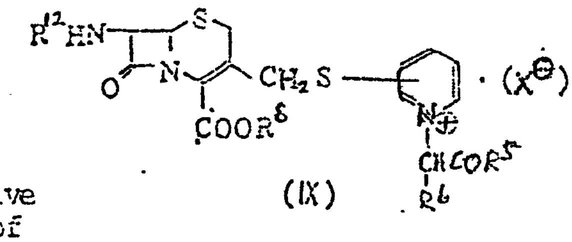

(III) or reactive derivative thereof (IX)

with α-(4-oxy-2-azetidinyl-substituted-imino)thiazolyl-acetic acid of formula (III) or reactive derivative thereof wherein $R^{12}$ represents a hydrogen atom or tri(lower-alkyl)silyl group, and the other symbols are as defined in claim 4.

or (3) reacting 7-(α-substituted-thiazolyl-α-(4-oxoazetidinyl-substituted-imino)acetamido-3-(acyloxy- or halogenomethyl)-$\Delta^3$-cephem-4-carboxylic acid derivative of formula (X) or salt thereof with 1-substituted-2 or 4-thioxopyridine or 1-substituted-3-mercaptopyridinium salt of formula (XI), and then, if necessary, releasing the protective group(s):

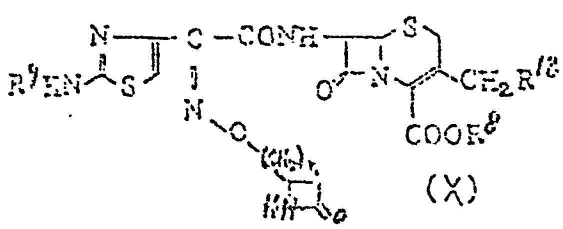

(X) or salt thereof

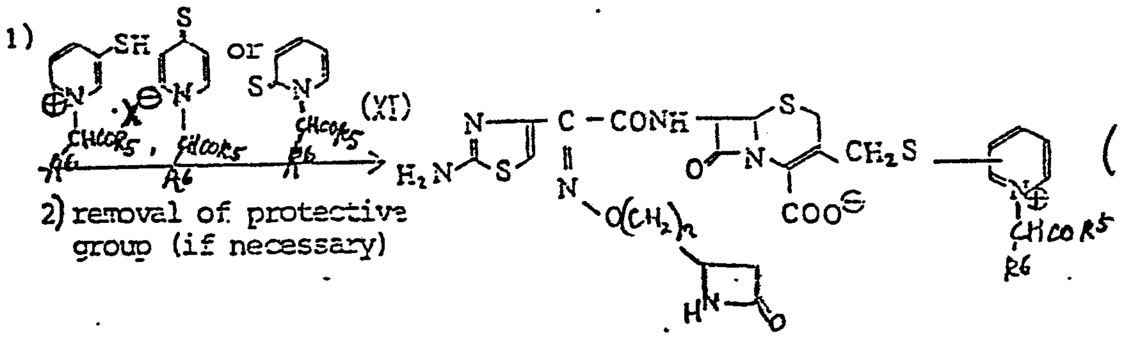


(Ia)

wherein $-COOR^8$ represents -COOH, -COO-protective group, or $-COO^-$; $(X^-)$ represents an anion when $-COOR^8$ is -COOH or -COO-protective group but but does not exist if $-COOR^8$ represents $-COO^-$; $R^{13}$ represents an acyloxy group or a halogen atom,and the other symbols are as defined in claim 4.

7. A method according to any of claims 4 to 6 which employs at least one reactant in salt form, and/or in which the product is subjected to a salt-forming reaction.

8. A pharmaceutical composition containing a compound according to claim 1, 2 or 3.

9. Starting and intermediate compounds as defined in any of claims 4 to 7.